# EUROPEAN PATENT SPECIFICATION

(11) **EP 3 794 622 B1**
(45) Date of publication and mention of the grant of the patent: **15.06.2022**
(21) Application number: 19730957.8
(22) Date of filing: 17.05.2019
(51) Int. Cl.: H01H 9/02, H04M 1/17

(54) **HYGIENICAL CASE FOR REMOTE CONTROLS**
HYGIENEHÜLLE FÜR FERNBEDIENUNGEN
ÉTUI HYGIÉNIQUE POUR TÉLÉCOMMANDES

(30) Priority: 18.05.2018 IT 201800005505
(43) Date of publication of application: 24.03.2021
(73) Proprietor: Ramonda, Luca, 33030 Moruzzo (UD) (IT); Mavridis, Georgios, 34135 Trieste (IT); Bellina, William, 33080 Procia (PN) (IT); Crapis, Massimo, 33100 Udine (IT)
(72) Inventor: Ramonda, Luca, 33030 Moruzzo (UD) (IT); Mavridis, Georgios, 34135 Trieste (IT); Bellina, William, 33080 Procia (PN) (IT); Crapis, Massimo, 33100 Udine (IT)
(74) Representative: Frare, Paolo
(86) International application number: PCT/IB2019/054087
(87) International publication number: WO 2019/220401

(56) References cited:
- EP-A1- 2 923 368
- WO-A1-2012/176035
- US-A- 4 819 265

## Description

The present invention refers to a hygienical case for remote controls.

Currently, in commonly used structures, i.e. adapted for being used by a plurality of users, like for example hotel rooms, hospitals, etc., it is known to use remote controls, wired or wireless, for remotely controlling electric or electronic devices, such as television sets, radios, bed tilting devices, curtain openers/closers, air conditioning systems, etc.

Such remote controls are typically handled by users that take turns in such communal structures, with the risk, if such remote controls are not properly cleaned or hygienised before being used by a subsequent user, of possible germs, bacteria, viruses or other pathogens that may be deposited on the remote control during the previous use, being transmitted to the next user. Known studies confirm that the area that has the greatest bacterial contamination in hotel rooms is indeed the television remote control.

In order to try to avoid such problems, there is for example the known Italian patent application number MO2012A0000282, corresponding to the European patent application n. EP 2 923 368 A1, which describes a case for remote controls comprising a coating wrapper for coating substantially the entire remote control provided with at least one keypad, the coating wrapper comprising at least one sector that is at least partially transparent and can be arranged at the keypad of the remote control to allow the keypad to be seen; the case comprises at least one covering element, which is removably associated with the coating wrapper substantially at the sector, and is adapted to prevent the keypad being seen until the covering element has been removed from the coating wrapper.

In the presence of the covering element, the user cannot see the keypad, for which reason he/she must remove it to be able to freely use the remote control; in this way, the user is certain that the region previously covered by the covering element has not been handled by a previous user.

The known solution described in the quoted Italian patent application does not, however, solve the problem of ensuring hygiene in the use of a remote control adapted for being used by a plurality of users; indeed, such a known solution ensures the hygiene only of the region of the wrapper over the keypad and protected by the covering element, whereas the rest of the wrapper remains subject to the risk of contamination.

Indeed, it is possible for a first user to handle the remote control, for example to move it, even without using it to control a device, and thus without having to remove the covering element; in this case, a second user that had to use the remote control, even by removing the covering element would still come into contact with possible germs, bacteria, viruses, or other pathogens deposited on the region of the wrapper not previously covered by the covering element, the removal of which would thus be ineffective in order to protect the second user. The risk of contamination is also accentuated by the fact that the area of the case not covered by the covering element must in any case be handled, for example by a cleaner of the building in which the remote control is used, to insert the remote control in the case itself.

The purpose of the present invention is to avoid the aforementioned drawbacks, and in particular to obtain a hygienical case for remote controls that can guarantee to the user of a remote control a use in hygienic conditions, even in the case in which such a case has already been handled previously.

Another purpose of the present invention is to provide a hygienical case for remote controls that is easy to use.

A further purpose of the present invention is to make a hygienical case for remote controls that can be used in combination with different types of remote control. These and other purposes according to the present invention are accomplished by making a hygienical case for remote controls, comprising a first wrapper adapted to cover totally, or substantially totally, a remote control, and a second wrapper fixed externally to the first wrapper so as to cover totally, or substantially totally, the same, such a second wrapper being permanently removable from the first wrapper. Advantageously, before the removal thereof, the second wrapper covers totally, or substantially totally, the first wrapper, in particular the external surface thereof, preferably like a "shell" or "skin".

The second wrapper, totally or substantially totally covering the first wrapper, in particular the external surface thereof, prevents the external surface of the first wrapper coming into contact with germs, viruses, bacteria or other pathogens before the removal of such a second wrapper.

The second wrapper, which completely covers the first, and which can be totally removed from the latter, ensures the perfect hygiene of the solution; indeed, irrespective of who might have previously handled the case, it is sufficient for a user to remove the second wrapper to be able to use the remote control, closed inside the first wrapper, in conditions of absolute hygiene. Indeed, the second wrapper, until it is removed, completely protects the first wrapper from germs, viruses, bacteria and other pathogens that the external surface of the second wrapper might have previously come into contact with, thus ensuring the total hygiene of the underlying first wrapper.

It should be emphasised that in the present patent application, the expression *"substantially totally cover"* is meant to indicate cover the totality of an object (for example a remote control, a wrapper), with the possible exclusion (for example due to holes or openings of very small size, in particular less than one millimetre) of regions thereof that are so small as not to constitute a risk for the passage of large amounts of germs, viruses, bacteria or possible pathogens.

Advantageously, before the removal thereof, the second wrapper totally covers the first wrapper like a "shell" or "skin".

Advantageously, the second wrapper cannot be fixed back onto the first wrapper after the second wrapper has been removed from the first wrapper.

In this way, the risk of the second wrapper being able to be removed and then reapplied to the first wrapper, for example by mistake, after it has been contaminated, is avoided.

Advantageously, the second wrapper can be fixed to the first wrapper by gluing and/or welding.

In a preferred embodiment, the second wrapper is made with a structure and/or from a material such that the removal thereof causes a permanent deformation and/or breaking and/or mechanical and/or aesthetic deterioration thereof, so as to make such a second wrapper unable to be reused.

In a preferred embodiment, the second wrapper can be made from a layer or film (or plurality of layers and/or films) that totally covers the external surface of the first wrapper (like a "shell" or "skin"); preferably, such a layer or film is fixed to the external surface of the first wrapper, for example by a removable adhesive, the removal of which preferably causes a permanent deformation and/or breaking and/or mechanical and/or aesthetic deterioration of the layer or film, so as to prevent it from being subsequently fixed back onto the first wrapper, or more generally reused.

In a further advantageous embodiment, the second wrapper can be fixed to the first wrapper by a connecting element. In this case, preferably, the permanent removal of the second wrapper from the first wrapper takes place by permanent breaking of the connecting element.

Preferably, the possible connecting element has a weakened structure, adapted for facilitating the breaking thereof.

In a preferred embodiment the possible connecting element is made in one piece (i.e. in a single body) with the first wrapper and the second wrapper. Preferably, the connecting element and the first and the second wrapper are made by co-moulding. Advantageously, the first wrapper is made from plastic material of suitable density so as not to allow germs or bacteria to pass.

Preferably, the first wrapper can be made from polyethylene (PE), or from another plastic material, of the biodegradable, recyclable non-biodegradable or non-recyclable type.

Advantageously, the second wrapper is made from plastic material of suitable density so as not to allow germs or bacteria to pass.

Preferably, the second wrapper can be made from polyethylene (PE), or another plastic material, of the biodegradable, recyclable non-biodegradable or non-recyclable type.

In a preferred embodiment, the second wrapper is impregnated/treated with a disinfecting substance, like for example the substance known by the trade name "AMUCHINA".

In a preferred embodiment, the case has a envelope-like configuration, more preferably having a rectangular plan, possibly with bevelled or rounded borders. Advantageously, the case has a flexible, and preferably, but not necessarily, elastically deformable structure, so as to better adapt to different types and sizes of remote control.

In a preferred embodiment, the first wrapper has a envelope-like configuration, more preferably having a rectangular plan, possibly with bevelled or rounded borders.

Preferably, the first wrapper has a flexible, and preferably, but not necessarily, elastically deformable structure, so as to better adapt to different types and sizes of remote control.

In a preferred embodiment, the second wrapper has a envelope-like configuration, more preferably having a rectangular plan, possibly with bevelled or rounded borders, arranged to totally, or substantially totally cover the first, like a "shell" or "skin".

Preferably, the second wrapper has a flexible, and preferably, but not necessarily, elastically deformable structure, so as to better adapt to different types and sizes of remote control.

In a further advantageous embodiment, the second wrapper comprises one or more layers or films fixed removably to the external surface of the first wrapper, completely cover the same like a "shell" or "skin". Advantageously, the case comprises an access opening for a remote control. Preferably, the case comprises a closing system adapted for totally, or substantially totally, closing such an access opening. In a preferred embodiment, such a closing system comprises an adhesive and/or a zipper, with or without a cursor. More preferably, such a closing system comprises a layer of adhesive positioned on an inner surface of the first wrapper, near the border of the access opening, adapted for allowing such a border to be fastened in closed position, so as to permanently close the access opening. Preferably, the case comprises a removable protective element of the layer of adhesive, adapted for preventing the closing by gluing of the access opening before the removal of such a removable protective element. Preferably, the removable protective element comprises a strip of plastic material.

In the advantageous case in which the case has a envelope-like structure, the access opening can preferably be formed at a perimeter end of such an envelope.

In a further advantageous embodiment, the second wrapper can be fixed to the first wrapper by means of a removable region of such a second wrapper, fixed to cover the external surface of the first wrapper, for example by means of a removable adhesive, or by welding, preferably at the border of the access opening.

In a preferred embodiment, the case comprises a gripping element, preferably positioned on said second wrapper, to facilitate the removal of the second wrapper from the first wrapper. More preferably, such a gripping element comprises a tongue that projects from the external surface of the second wrapper. Advantageously, the first wrapper comprises a transparent region adapted to lay over a keypad of a remote control inserted in it.

Preferably, but not necessarily, the first wrapper is totally transparent.

In a preferred embodiment, the second wrapper comprises at least one obstructing region at least partially laying over the transparent region of the first wrapper, so as to at least partially conceal the keypad of a remote control inserted in the case and/or make it not freely usable while the second wrapper is fixed to the first wrapper.

In a further advantageous embodiment, the possible obstructing region of the second wrapper is opaque or partially opaque, so as to at least partially conceal the keypad of a remote control inserted in the case and/or make it not freely usable while the second wrapper is fixed to the first wrapper.

Preferably, the possible obstructing region of the second wrapper comprises one or more inscriptions and/or images positioned on it, so as to at least partially conceal the keypad of a remote control inserted in the case and/or make it not freely usable while the second wrapper is fixed to the first wrapper. Preferably, the possible one or more inscriptions and/or images contain at least one instruction concerning the removal of the second wrapper.

In a further advantageous embodiment, the possible obstructing region of the second wrapper has an increased rigidity and/or thickness, so as to make the keypad of a remote control inserted in the case not freely usable while the second wrapper is fixed to the first wrapper.

The characteristics and advantages of the hygienical case for remote controls according to the present invention will become clearer from the following description, given as an example and not for limiting purposes, referring to the attached schematic drawings, in which:
- figure 1 is a schematic plan view of a remote control and of a case according to the invention before the insertion of the remote control in the case;
- figure 2 is a side view of the remote control and of the case of figure 1;
- figure 3 is a side view of the remote control and of the case of figure 1 during the insertion of the remote control in the case;

- figure 3b is an enlarged detail of figure 3;
- figure 4 is a side view of the remote control and of the case of figure 1, with the remote control totally inserted in the case;
- figure 5 is a side view of the remote control and of the case of figure 1 during the removal of the second wrapper from the first wrapper;
- figure 6 is a side view of the remote control and of the case of figure 1 during a further step of the removal of the second wrapper from the first wrapper;
- figure 7 is a side view of the remote control inserted in the case of figure 1 with the second wrapper totally removed;
- figure 8 is a side view of the remote control and of the case of figure 1 during the use of the remote control;
- figure 9 is a schematic plan view of a wired remote control inserted in a case according to the invention with the second wrapper removed;
- figure 10 is a side view of the remote control and of the case of figure 9;
- figure 11 is a schematic plan view of a remote control inserted in a further embodiment of a case according to the invention;
- figure 12 is a schematic plan view of a remote control inserted in a further embodiment of a case according to the invention, before the removal of the second wrapper;
- figures 13, 14 and 15 are schematic plan views of the remote control and of the case of figure 12, in various steps of the removal of the second wrapper;
- figure 16 is a schematic plan view of a further variant embodiment of a case according to the invention.

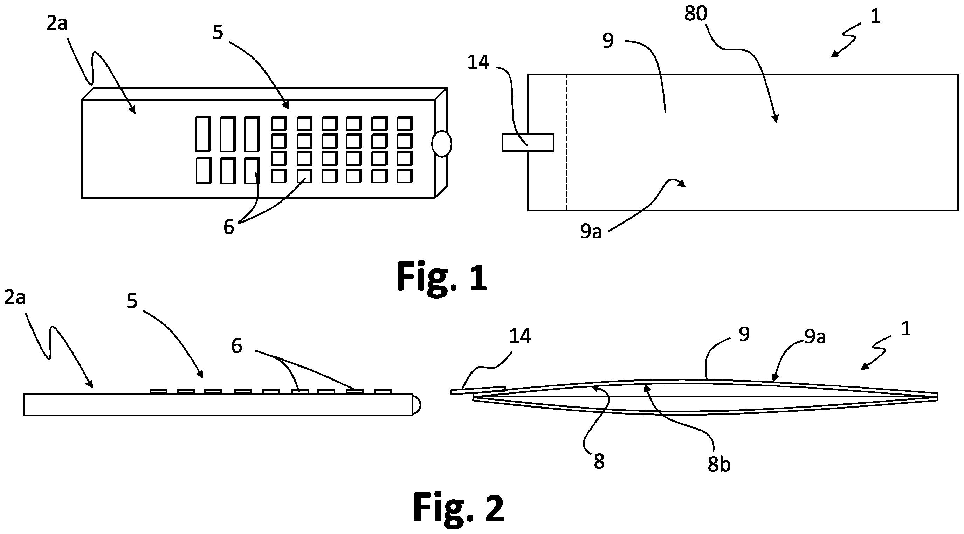

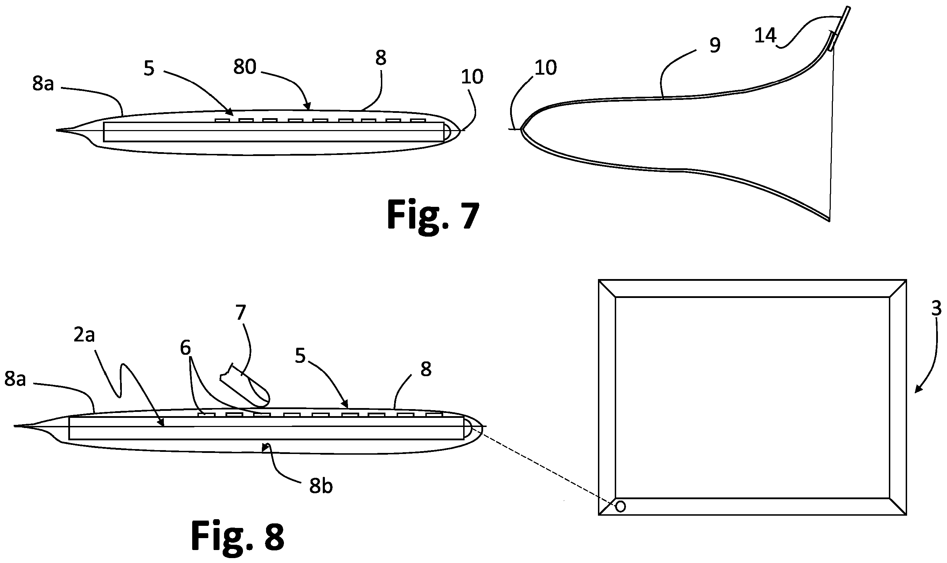

With reference to the attached figures, a hygienical case for remote controls is wholly indicated with numeral 1; figures 1 to 8, and 11 to 15, represent a first example of remote control, indicated with numeral 2a, of the wireless type, able to be used to remotely control an electric or electronic device. In the nonlimiting example of figure 8, the electronic device able to be controlled remotely by the remote control 2a is a television set 3, but in a different embodiment, not represented, it could for example be a radio, a computer, a tilting bed, a device for opening/closing curtains, for controlling air conditioners, etc.

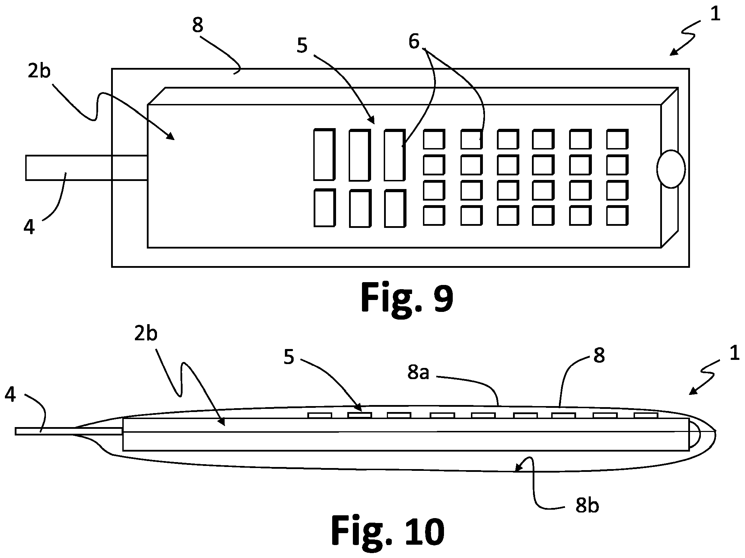

In a further embodiment, illustrated for example in figures 9 and 10, the remote control, indicated with numeral 2b, can advantageously be of the wired type, i.e. equipped with a cable 4 that connects it to an electric or electronic device, not represented, to be controlled through the remote control 2b.

In any case, it is emphasised that the invention can be used both with a wireless remote control and with a wired remote control.

The remote control, both in its wireless version 2a, and in its wired version 2b, preferably comprises a keypad 5, comprising one or more keys 6, preferably of the electromechanical and/or "touch" type, by acting on which, for example pressing them, a user, whose finger 7 is represented schematically in figure 8, can activate one or more functions of the device controlled remotely by the remote control; for example, in the case in which such a device is a television set 3, the keypad 5 can allow the channel to be changed, the volume to be increased, etc.

In the case in which, for example, the remote control is intended for controlling the tilt of a bed, the keypad 5 can comprise keys for controlling such tilt. The case 1 comprises a first wrapper 8 adapted for totally, or substantially totally, housing the remote control 2a, or 2b.

Advantageously, the first wrapper 8 comprises at least one transparent region 80 adapted for laying over the keypad 5 of a remote control 2a, 2b inserted in it, so as to allow it to be seen by a user.

Advantageously, but not necessarily, the first wrapper is completely transparent.

The case 1 comprises a second wrapper 9 fixed externally to the first wrapper 8 so as to totally, or substantially totally, cover the same, and permanently removable from such a first wrapper 8.

Advantageously, before the removal thereof, the second wrapper 9 totally covers the first wrapper 8, in particular the external surface 8a thereof, like a "shell" or "skin".

Advantageously, as will be explained better hereinafter, the second wrapper 9 cannot be fixed back to the first wrapper 8 once the second wrapper 9 has been removed from the first wrapper 8.

Advantageously, the first wrapper 8 is made from plastic material of suitable density so as not to allow germs or bacteria to pass (for example it can have a thickness comprised between 0.005 mm and 1 mm); preferably the first wrapper 8 is made from polyethylene (PE), or another plastic material, of the biodegradable, recyclable non-biodegradable or non-recyclable type.

Advantageously, the second wrapper 9 is also made from plastic material of suitable density so as not to allow germs or bacteria to pass (for example it can have a thickness comprised between 0.005 mm and 1 mm), preferably polyethylene (PE), or another plastic material, of the biodegradable, recyclable non-biodegradable or non-recyclable type. Advantageously, but not necessarily, the second wrapper 9 is impregnated or treated with a disinfecting substance, like for example the substance known by the trade name "AMUCHINA".

In a preferred embodiment, the second wrapper 9 is fixed to the first wrapper 8 by gluing and/or welding.

For example, the second wrapper 9 can be made from a layer or film that totally covers the external surface 8a of the first wrapper 8 (like a "shell" or "skin"); such a layer or film is fixed to such an external surface 8a, for example, through a removable adhesive, the removal of which causes a permanent deformation and/or breaking and/or mechanical and/or aesthetic deterioration of the layer or film itself, so as to prevent it from subsequently being fixed back to the first wrapper 8, or, more generally, reused.

In a further advantageous embodiment, represented for example in figures 1 to 8, the second wrapper 9 can be fixed to the first wrapper 8 by a connecting element 10. Advantageously, the connecting element 10 can be provided, alternatively or in combination, with a further fixing means, like for example gluing.

For example, the second wrapper 9 can be made from a layer or film that totally covers the external surface 8a of the first wrapper 8, such a layer or film being fixed to such an external surface 8a through a removable adhesive, and being further fixed to the first wrapper 8 by a connecting element 10, for example a flap of plastic material that joins the first wrapper 8 to the second wrapper 9.

Preferably, the connecting element 10 joins the external surface 8a of the first wrapper 8 to the inner surface 9b of the second wrapper 9, and is positioned so as to be totally covered by the second wrapper 9 when it totally covers the first wrapper 8, so that the second wrapper 9 also protects the connecting element 10 from contact with germs, viruses, bacteria, or other pathogens that might come into contact with the external surface 9a of the second wrapper 9 before it has been removed.

Advantageously, the permanent removal of the second wrapper 9 from the first wrapper 8 takes place through permanent breaking of the connecting element 10.

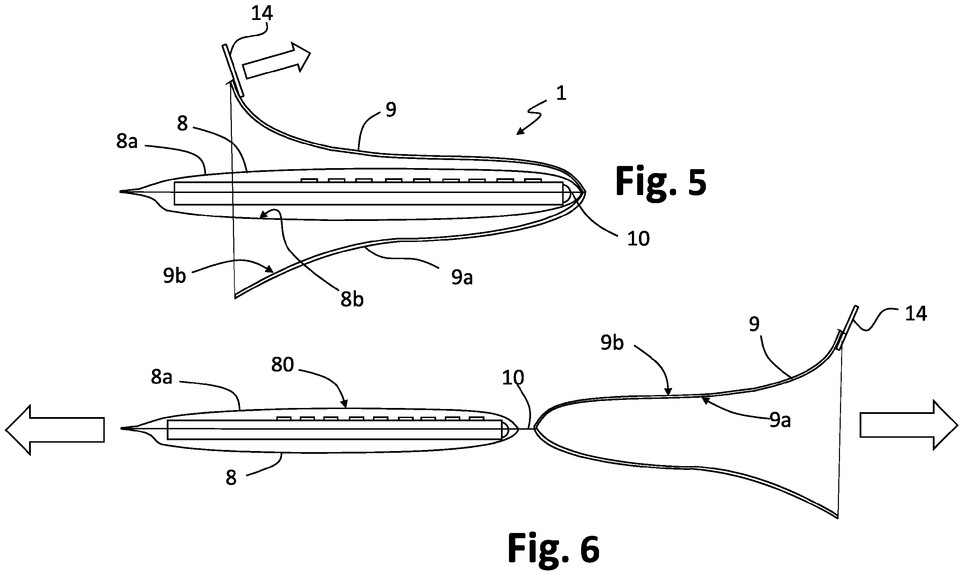

For example, with reference to figures 6 and 7, by exerting a traction of the second wrapper 9 in the direction away from the first wrapper 8, in order to permanently remove such a second wrapper 9 from the first wrapper 8, once the ultimate tensile strength of the connecting element 10 has been exceeded, the latter breaks, with consequent permanent removal of the second wrapper 9 from the first wrapper 8.

Preferably, but not necessarily, the connecting element 10 has a weakened structure, adapted to facilitate the breaking thereof; for example, the connecting element 10 can have tear perforations obtained with a series of aligned holes that pass through it, reducing the resistance thereof to tearing.

Preferably, but not necessarily, the connecting element 10 is made in one piece with the first wrapper 8 and with the second wrapper 9, for example through co-moulding of such three elements, or through over-moulding of the connecting element 10 on the first and second wrapper, to connect them together. Advantageously, the case 1 has a envelope-like configuration, preferably with a rectangular plan, possibly with bevelled or rounded borders.

Preferably, the case 1 has a flexible, and advantageously, but not necessarily, elastically deformable structure, so as to better adapt to different types and sizes of remote control.

In a preferred embodiment, the first wrapper 8 has a envelope-like configuration, more preferably with a rectangular plan, possibly with bevelled or rounded borders.

Preferably the first wrapper 8 has a flexible, and preferably, but not necessarily, elastically deformable structure, so as to better adapt to different types and sizes of remote control.

In a preferred embodiment, the second wrapper 9 has a envelope-like configuration, more preferably with a rectangular plan, possibly with bevelled or rounded borders, arranged to totally, or substantially totally cover the first wrapper, like a "shell" or "skin". Preferably, the second wrapper 9 has a flexible, and preferably, but not necessarily, elastically deformable structure, so as to better adapt to different types and sizes of remote control.

In an advantageous variant embodiment, not represented in the attached figures, the second wrapper 9 can comprise one or more layers or films removably fixed to the external surface 8a of the first wrapper 8, to totally, or substantially totally, cover the same. Advantageously, the case 1 comprises an access opening 11 for the insertion of the remote control 2a, 2b.

In the case in which the case 1 has a envelope-like structure, the access opening 11 can be advantageously formed at a perimeter end of such an envelope.

In a further advantageous embodiment, not represented, the access opening 11 can be formed in a different position of the case 1.

Preferably, the case 1 comprises a closing system adapted for totally, or substantially totally, closing the access opening 11.

It is emphasised that in the present patent application, the expression *"substantially totally close the access opening"* means completely closing the opening, with the possible exclusion of regions thereof that are so small (preferably of maximum dimensions of less than one millimetre) as not to constitute a risk for the passage of large amounts of germs, viruses, bacteria or possible pathogens.

Advantageously, the closing system of the access opening 11 can comprise an adhesive and/or a zipper, of the type with or without a cursor.

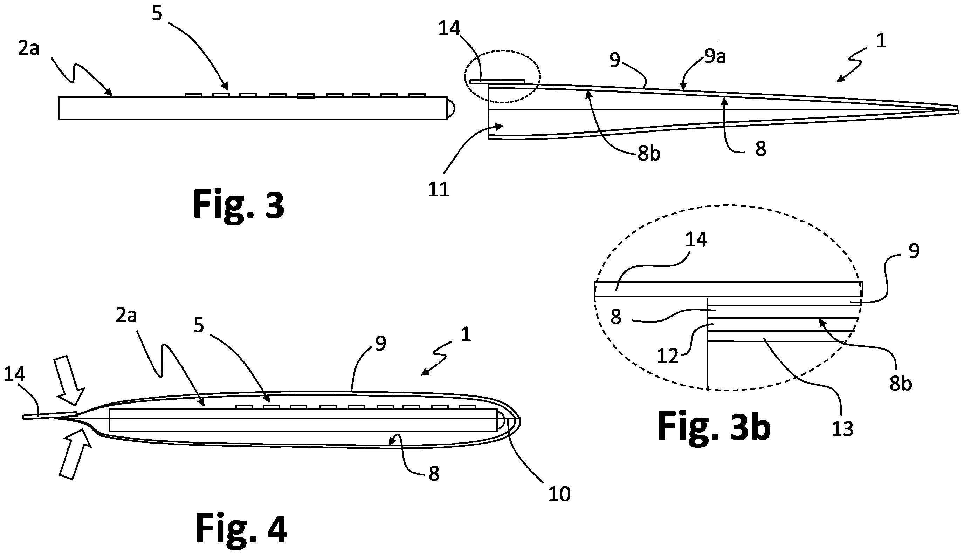

For example, in the example embodiment illustrated schematically in figure 3b, such a closing system can advantageously comprise a layer of adhesive 12 positioned on an inner surface 8b of the first wrapper 8, near to the border of the access opening 11; the layer of adhesive 12 allows such a border to be fastened in closed position (as shown for example in figure 4), so as to permanently close the access opening 11.

Advantageously, the case 1 can comprise a removable protective element 13 of the layer of adhesive 12, adapted to prevent the closing by gluing of the access opening 11 before the removal of such a removable protective element 13.

Advantageously, the removable protective element 13 can comprise a strip of plastic material.

In the example embodiment illustrated in figures 9 and 10, the border of the access opening 11, thanks to the flexibility of the case 1, can be closed on itself to wrap around the cable 4, allowing it to come out from the case 1 and at the same time ensuring that the remote control 2b is totally, or substantially totally, closed in the case 1.

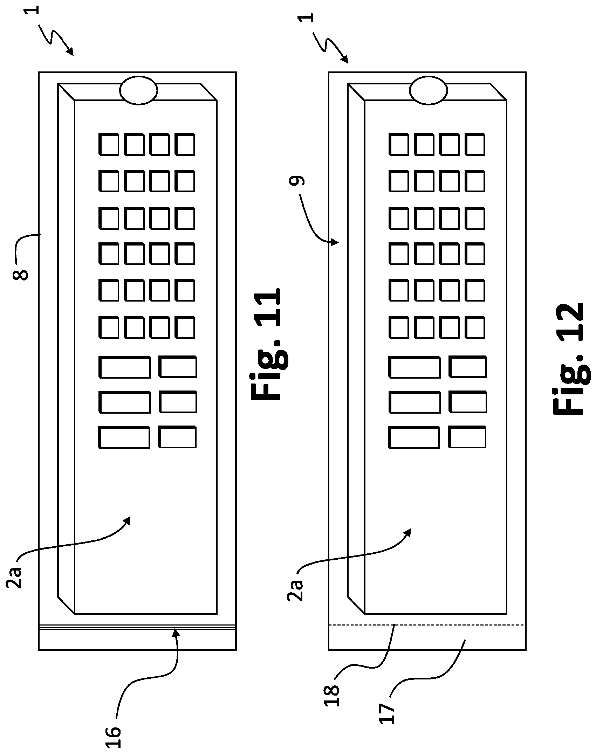

In a further embodiment, represented schematically in figure 11, the closing system of the access opening 11 can comprise a zipper 16 arranged at such an access opening 11 on the first wrapper 8, or any known fixing means. In the example embodiment of figure 11, the zipper 16 is not provided with a cursor, but in a further advantageous embodiment, not represented, the zipper 16 can be provided with a cursor, adapted to facilitate the closing thereof.

In a further advantageous embodiment, represented schematically in figures 12 to 15, the second wrapper 9 can be fixed to the first wrapper 8 through a removable region 17 of such a second wrapper, fixed to cover the external surface 8a of the first wrapper 8, for example through a removable adhesive, advantageously at the border of the access opening 11.

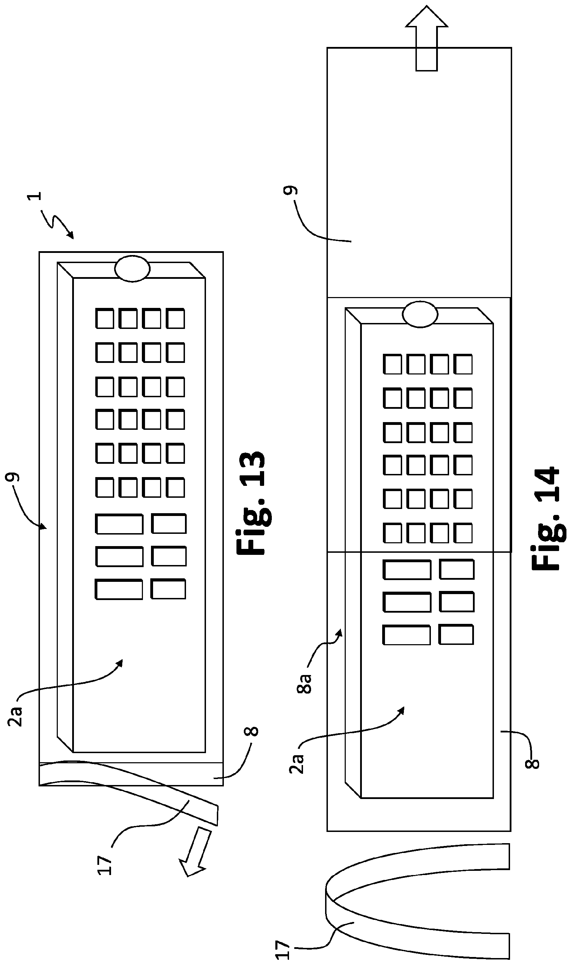

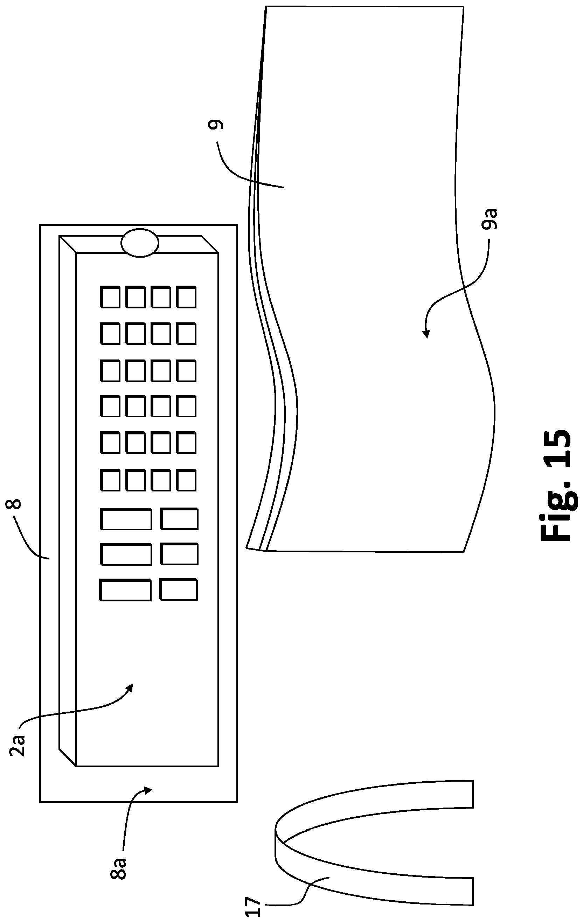

As illustrated schematically in figures 12 to 15, in such an advantageous embodiment, once a remote control, for example of the wireless type 2a (but the same is also valid in the case of a wired remote control 2b), has been inserted in the case 1, and the access opening 11 thereof has been closed through the closing system provided (for example an adhesive and/or a zipper, with or without a cursor) the removable region 17 of the second wrapper 9 can be removed from the rest of the second wrapper 9 itself, for example tearing it at a weakened region 18, and separating it from the external surface 8a of the first wrapper 8, for example by pulling. At this point, as illustrated for example in figures 14 and 15, the second wrapper 9 can be removed, leaving the remote control 2a protected by the first wrapper 8.

Advantageously, the case 1 can comprise a gripping element adapted to facilitate the removal of the second wrapper 9 from the first wrapper 8.

Preferably, the gripping element comprises a tongue 14 that projects from the external surface 9a of the second wrapper 9.

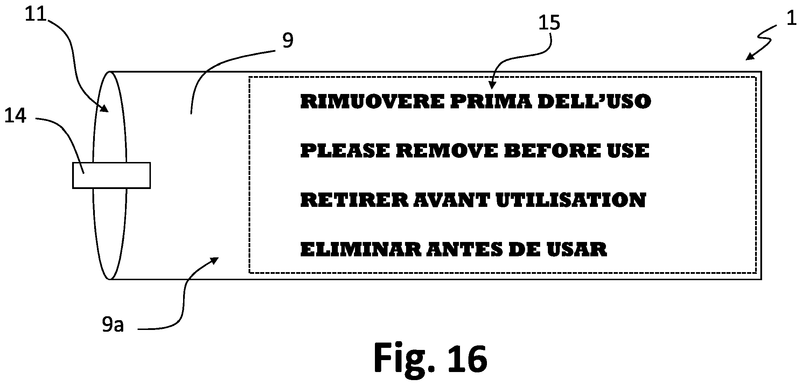

In an advantageous variant embodiment, represented for example in figure 16, the second wrapper 9 comprises at least one obstructing region 15 at least partially laying over the transparent region 80 of the first wrapper 8, so as to at least partially conceal the keypad 5 of a remote control 2a, 2b inserted in the case 1 and/or make it not freely usable while such a second wrapper 9 is fixed to the first wrapper 8.

In an advantageous embodiment, the obstructing region 15 of the second wrapper 9 is opaque or partially opaque, so as to at least partially conceal the keypad 5 of a remote control 2a, 2b inserted in the case 1 and/or make it not freely usable while such a second wrapper 9 is fixed to the first wrapper 8.

Preferably, but not necessarily, the obstructing region 15 comprises one or more inscriptions and/or images positioned on the same, so as to at least partially conceal the keypad 5 of a remote control 2a, 2b inserted in the case 1 and/or make it not freely usable while such a second wrapper 9 is fixed to the first wrapper 8.

Preferably, such one or more inscriptions and/or images contain at least one instruction concerning the removal of the second wrapper 9; such instructions can comprise, for example, the indication that the second wrapper 9 must be removed before the remote control is used.

In a further advantageous embodiment, the obstructing region 15 has an increased rigidity and/or thickness, so as not to make the keypad 5 of a remote control 2a, 2b inserted in the case 1 freely usable (for example because the increased thickness of the obstructing region 15 makes the keypad unable to be freely handled) while the second wrapper 9 is fixed to the first wrapper 8; such increased rigidity and/or thickness can be obtained, for example, through the positioning of an insert made of rigid plastic material inside or above the second wrapper 9.

In a further advantageous variant embodiment, not represented, irrespective of whether there is an obstructing region 15, the first wrapper 8 and/or the second wrapper 9 can carry one or more inscriptions and/or drawings, for example showing a trade name or logo and/or operating instructions for using the case 1, like for example the instructions of removing the second wrapper 9 before use, or the indication to request a new case 1 in the case in which the second layer is not present (for example because it has already been removed by a previous user).

The operation of the case 1 according to the invention is as follows.

Whenever the structure at which the remote control is installed is cleaned and prepared to receive a new user, the person tasked with cleaning and/or preparation removes a possible case already present on a remote control 2a, 2b, and inserted the latter in a case 1 according to the invention, through the access opening 11, making sure that the remote control 2a, 2b is totally, or substantially totally, closed in the case 1.

The access opening 9 is then closed through the closing system present in the case 1, for example, with reference to figures 1-10, removing the protective element 13 and then closing the border of the access opening 11 on the layer of adhesive 12, so as to seal the border of the access opening 11 in closed condition. In the further embodiment illustrated in figure 11 the access opening can be closed by acting on the zipper 16.

In such a condition, the remote control 2a, 2b is totally closed by the case 1; in the case in which the remote control 2b is of the wired type, only the cable 4 comes out from the case 1.

When a new user is about to use the remote control 2a, 2b closed in the case 1, before using it removes, for example by acting on the gripping element 14, the second wrapper 9, which previously totally closed the first wrapper 8 and the remote control 2a, 2b.

Once removed, the second wrapper 9 cannot be fixed back to the first wrapper 8, for example because it has deteriorated during its removal and/or because a possible connecting element 10 that joined it to the first wrapper 8 has been broken during the removal of the second wrapper 9.

Before the removal of the second wrapper 9, the external surface 8a of the underlying first wrapper 8 was protected by the presence of the second wrapper 9, so that possible handling of the case prior to the removal of the second wrapper 9 does not entail the risk of the external surface 8a of the first wrapper 8 coming into contact with possible germs, bacteria, viruses, or other pathogens.

In the advantageous, but not necessary, case in which the second wrapper 9 has an obstructing region 15, the user is forced to remove the second layer 9 to be able to freely use the remote control; in this way, the risk of the user forgetting to remove the second layer 9 before use is reduced.

From the description that has been made the characteristics of the device object of the present invention are clear, just as the relative advantages are also clear.

Finally, it is clear that the device thus conceived can undergo numerous modifications and variants, all encompassed by the scope of protection, which is defined by the appended claims; moreover, all of the details can be replaced by technically equivalent elements. In practice, the materials used, as well as the sizes, can be whatever according to the technical requirements.

## Claims

1. Hygienical case (1) for remote controls, comprising a first wrapper (8) adapted to cover totally, or substantially totally, a remote control (2a, 2b),
**characterized by** comprising a second wrapper (9) fixed externally to said first wrapper (8) so as to cover totally, or substantially totally, the first wrapper (8), wherein the second wrapper (9) is permanently removable from the first wrapper (8) .

2. Case (1) according to claim 1, **characterized in that** said second wrapper (9) is fixed to said first wrapper by gluing and/or welding.

3. Case (1) according to any of the claims 1 or 2, **characterized in that** said second wrapper (9) is fixed to said first wrapper (8) by a connecting element (10).

4. Case (1), according to claim 3, **characterized in that** the permanent removal of said second wrapper (9) from said first wrapper (8) takes place by permanent breaking of said connecting element (10).

5. Case (1), according to one or more of the preceding claims, **characterized in that** said second wrapper (9) is impregnated/treated with a disinfecting substance.

6. Case (1), according to one or more of the preceding claims, **characterized in that** said second wrapper (9) comprises one or more layers or films fixed removably to the external surface (8a) of said first wrapper (8), to completely cover the same.

7. Case (1), according to one or more of the preceding claims, **characterized in that** it comprises an access opening (11) for a remote control (2a, 2b).

8. Case (1), according to claim 7, **characterized in that** it comprises a closing system (12) adapted to totally, or substantially totally, close said access opening (11).

9. Case (1), according to claim 8, **characterized in that** said closing system comprises a layer of adhesive (12) positioned on an inner surface (8b) of the first wrapper (8), near the border of said access opening (11), adapted to allow said border to be fastened in a closed position, so as to permanently close said access opening (11), or a zipper (16), with or without a cursor.

10. Case (1), according to one or more of the preceding claims, **characterized in that** it comprises a gripping element (14) to facilitate the removal of said second wrapper (9) from said first wrapper (8).

## Patentansprüche

1. Hygienisches Gehäuse (1) für Fernbedienungen, mit einer ersten Hülle (8), die geeignet ist, eine Fernbedienung (2a, 2b) vollständig oder im Wesentlichen vollständig abzudecken,
**dadurch gekennzeichnet, dass sie** eine zweite Umhüllung (9) umfasst, die außen an der ersten Umhüllung (8) befestigt ist, um die erste Umhüllung (8) vollständig oder im Wesentlichen vollständig abzudecken, wobei die zweite Umhüllung (9) dauerhaft von der ersten Umhüllung (8) entfernbar ist.

2. Gehäuse (1) nach Anspruch 1, **dadurch gekennzeichnet, dass** die zweite Umhüllung (9) an der ersten Umhüllung durch Kleben und/oder Schweißen befestigt ist.

3. Gehäuse (1) nach einem der Ansprüche 1 oder 2, **dadurch gekennzeichnet, dass** die zweite Umhüllung (9) an der ersten Umhüllung (8) durch ein Verbindungselement (10) befestigt ist.

4. Gehäuse (1) nach Anspruch 3, **dadurch gekennzeichnet, dass** das dauerhafte Entfernen der zweiten Umhüllung (9) von der ersten Umhüllung (8) durch dauerhaftes Brechen des Verbindungselements (10) erfolgt.

5. Gehäuse (1) nach einem oder mehreren der vorhergehenden Ansprüche, **dadurch gekennzeichnet, dass** die zweite Umhüllung (9) mit einer desinfizierenden Substanz imprägniert/behandelt ist.

6. Gehäuse (1) nach einem oder mehreren der vorhergehenden Ansprüche, **dadurch gekennzeichnet, dass** die zweite Umhüllung (9) eine oder mehrere Schichten oder Folien umfasst, die abnehmbar an der Außenfläche (8a) der ersten Umhüllung (8) befestigt sind, um diese vollständig abzudecken.

7. Gehäuse (1) nach einem oder mehreren der vorhergehenden Ansprüche, **dadurch gekennzeichnet, dass** es eine Zugangsöffnung (11) für eine Fernsteuerung (2a, 2b) aufweist.

8. Gehäuse (1) nach Anspruch 7, **dadurch gekennzeichnet, dass** es ein Verschlusssystem (12) umfasst, das geeignet ist, die Zugangsöffnung (11) vollständig oder im Wesentlichen vollständig zu verschließen.

9. Gehäuse (1) nach Anspruch 8, **dadurch gekennzeichnet, dass** das Verschlusssystem eine Klebstoffschicht (12) umfasst, die auf einer Innenfläche (8b) der ersten Hülle (8) in der Nähe des Randes der Zugangsöffnung (11) positioniert ist und geeignet ist, das Befestigen des Randes in einer geschlossenen Position zu ermöglichen, um die Zugangsöffnung (11) dauerhaft zu verschließen, oder einen Reißverschluss (16) mit oder ohne Läufer.

10. Gehäuse (1) nach einem oder mehreren der vorhergehenden Ansprüche, **dadurch gekennzeichnet, dass** es ein Greifelement (14) umfasst, um das Entfernen der zweiten Hülle (9) von der ersten Hülle (8) zu erleichtern.

## Revendications

1. Boîtier hygiénique (1) pour télécommandes, comprenant une première enveloppe (8) conçue pour recouvrir totalement, ou sensiblement totalement, une télécommande (2a, 2b),
**caractérisé en ce qu'il** comprend une seconde enveloppe (9) fixée à l'extérieur de ladite première enveloppe (8) de manière à recouvrir totalement, ou sensiblement totalement, la première enveloppe (8), dans lequel la seconde enveloppe (9) peut être retirée de manière permanente de la première enveloppe (8).

2. Boîtier (1) selon la revendication 1, **caractérisé en ce que** ladite seconde enveloppe (9) est fixée à ladite première enveloppe par collage et/ou soudage.

3. Boîtier (1) selon l'une quelconque des revendications 1 ou 2, **caractérisé en ce que** ladite seconde enveloppe (9) est fixée à ladite première enveloppe (8) par un élément de liaison (10).

4. Boîtier (1) selon la revendication 3, **caractérisé en ce que** le retrait permanent de ladite seconde enveloppe (9) de ladite première enveloppe (8) a lieu par rupture permanente dudit élément de liaison (10).

5. Boîtier (1) selon une ou plusieurs des revendications précédentes, **caractérisé en ce que** ladite seconde enveloppe (9) est imprégnée/traitée avec une substance désinfectante.

6. Boîtier (1) selon une ou plusieurs des revendications précédentes, **caractérisé en ce que** ladite seconde enveloppe (9) comprend une ou plusieurs couches ou films fixés de manière amovible à la surface externe (8a) de ladite première enveloppe (8), pour la recouvrir complètement.

7. Boîtier (1) selon une ou plusieurs des revendications précédentes, **caractérisé en ce qu'**il comporte une ouverture d'accès (11) pour une télécommande (2a, 2b).

8. Boîtier (1) selon la revendication 7, **caractérisé en ce qu'**il comprend un système de fermeture (12) conçu pour fermer totalement, ou sensiblement totalement, ladite ouverture d'accès (11).

9. Boîtier (1) selon la revendication 8, **caractérisé en ce que** ledit système de fermeture comprend une couche d'adhésif (12) positionnée sur une surface intérieure (8b) de la première enveloppe (8), près du bord de ladite ouverture d'accès (11), conçue pour permettre audit bord d'être fixé dans une position fermée, de manière à fermer de façon permanente ladite ouverture d'accès (11), ou une fermeture à glissière (16), avec ou sans curseur.

10. Boîtier (1) selon une ou plusieurs des revendications précédentes, **caractérisé en ce qu'**il comprend un élément de préhension (14) pour faciliter le retrait de ladite seconde enveloppe (9) de ladite première enveloppe (8).
